(19) Europäisches Patentamt
European Patent Office
Office européen des brevets

(11) **EP 1 903 954 B1**

(12) **EUROPEAN PATENT SPECIFICATION**

(45) Date of publication and mention
of the grant of the patent:
**09.12.2009 Bulletin 2009/50**

(51) Int Cl.:
**A61B 17/16** *(2006.01)*     **A61B 17/32** *(2006.01)*
**A61C 3/02** *(2006.01)*

(21) Application number: **06779922.1**

(22) Date of filing: **17.07.2006**

(86) International application number:
**PCT/IB2006/002118**

(87) International publication number:
**WO 2007/010389 (25.01.2007 Gazette 2007/04)**

(54) **SURGICAL BUR WITH ANTI-CHATTER FLUTE GEOMETRY**

CHIRURGISCHE FRÄSE MIT ANTI-CHATTER-KEHLGEOMETRIE

FRAISE CHIRURGICALE À GÉOMÉTRIE À GOUJURES ANTI-BROUTAGE

(84) Designated Contracting States:
**DE FR GB IE IT**

(30) Priority: **19.07.2005 US 700384 P**

(43) Date of publication of application:
**02.04.2008 Bulletin 2008/14**

(73) Proprietor: **Stryker Ireland Limited**
**Carrigtwohill, County Cork (IE)**

(72) Inventor: **O'DONOGHUE, Denis**
**Killarney,**
**Country Kerry (IE)**

(74) Representative: **Röthinger, Rainer**
**Wuesthoff & Wuesthoff**
**Patent- und Rechtsanwälte**
**Schweigerstrasse 2**
**81541 München (DE)**

(56) References cited:
**DE-A1- 3 032 493     DE-C1- 19 718 938**
**DE-C1- 19 826 276     GB-A- 342 881**
**US-B1- 6 257 889**

**Description**

FIELD OF THE INVENTION

This invention is generally related to surgical burs. More particularly, this invention is related to a surgical bur with a bur head geometry that substantially reduces chatter when the bur is applied to a surgical site.

BACKGROUND OF THE INVENTION

A tool used to perform a surgical procedure is the bur. A bur generally consists of a head formed from rigid material, typically metal, shaped to have a number of flutes. The flutes are formed to define tissue cutting edges. A shaft extends rearwardly from the head. The free end of the shaft has a feature that facilitates locking the shaft to a powered handpiece. The actuation of the handpiece results in the rotation of the bur. During a surgical procedure, the bur head is placed against a surgical site where a section of tissue is to be removed. The rotating cutting edges excise tissue away from the surgical site. Burs of various shapes and sizes are used in procedures such as orthopedic surgery, neuro and spinal surgery, ear noise and throat surgery and in other surgical procedures in which a sub-procedure is to selectively remove a section of tissue.

Burs work well for the purposes for which they are designed. Nevertheless, a problem associated with some burs is chatter. Chatter is the back and forth vibration of a bur head against the surface to which the bur head is applied. Chatter occurs as a result of bur's individual cutting edges repeatedly being forced against the tissue against which the bur head is applied. Generally, there three reasons a bur may start to chatter.

One reason a bur starts to chatter is because it receives an input of energy due to a process known as regeneration of waviness. This process is due to the fact that when a cutting edge passes across a section of tissue, it leaves a specific wavy (essentially sinusoidal) profile along the surface of the tissue. If two adjacent cutting edges cut in phase, the second cutting edge excises tissue along a surface profile identical to that along which in was excised by the first flute. In practice, due to the invariable movements of the bur head and the tissue, this does not happen. When any two successive cutting edges pass over the same tissue section, the second flute cutting edge removes tissue on a path that does not overlap the tissue wave excised by the first cutting edge. Consequently, the debris chips cut by the second cutting edge have variable thickness. This means, during the process in which the second cutting edge excises the chip from the tissue, the cutting edge and its flute are subjected to variable forces. Over time, the repetitive exposure of the bur flutes to these variable forces causes the bur to undergo forced vibration.

A second reason a bur may chatter is that it is rotated at its resonant frequency. If this occurs, the repetitive force against the flutes self-excites the bur to move back and forth through a continually increasing range of motion.

The third reason a bur may chatter is due to the depth of the cut in the tissue against which the bur head is applied. If a bur head is pressed against the tissue so as to make only a relatively shallow cut, the overall time any two adjacent flutes are exposed to the tissue being cut is relatively low. The time in which the two adjacent flutes, as well as the spatial gap between the flutes, are exposed to the open environment is relatively high. During these relatively long time periods, tissue cut from bur and entrained in this gap is able to be discharged away from the bur head. This gap is then relatively debris-free the next time it rotates against the in-place tissue. Additional newly excised tissue fills this gap. However, if the bur head is pressed against the tissue to make a deep cut, the time in which the spatial gap between any two flutes is located against the in-place tissue increases. The time this gap is exposed to the open environment drops. Consequently, there may not be enough time for tissue entrained in this gap to be discharged. These gaps between the flutes clog. If this occurs, during the next time period in which the gap is rotated against the in-place. It is believed that that build up of chips between the flutes and their cutting edges clogs the bur head. This clogging, in turn, it is believed causes a forced vibration and the resultant chatter.

Surgical burs for dental purposes are known from GB 342,881 A, from which the preamble of claim 1 emanates, DE 198 26 276 C1 as well as DE 197 18 938 C1.

SUMMARY OF THIS INVENTION

This invention is directed to the design of a new and useful surgical bur according to the preamble of claim 1. The bur of this invention is provided with cutting edges positioned to reduce, if not eliminate, chatter that occurs during the use of the bur according to the characterizing portion of claim 1.

The bur of this invention has plural sets of flutes. Each flute defines a cutting edge, an edge that removes tissue from the surgical site to which the bur is applied. The flutes in a first set of flutes are shaped to have cutting edges that emerge from the body of the bur head at a first position relative to the distal end tip of bur head. Often, but not always, these cutting edges emerge from the distal end tip. The flutes in the second set of flutes are shaped to have cutting edges that emerge from the bur head body at a second position spaced proximally from the first position.

The distal ends of the second set of flutes are chamfered. As a result of the chamfers, the cutting edges of the flutes start at positions proximal to the distal ends of the flutes.

In some versions of the invention, the bur head is formed so that the second set of flutes emerges from the bur head at locations proximal to the locations from which the first set emerges. Preferred embodiments are set forth in the dependent claims.

BRIEF DESCRIPTION OF THE DRAWINGS

The invention is pointed out with particularity in the claims. The above and further features of this invention may be better understood by reference to the following detailed description taken in conjunction with the accompanying drawings in which:

Figure 1 is a side view of a basic surgical bur constructed in accordance with this invention;

Figure 1A is a side view of an the surgical bur of this invention illustrating an alternative geometric feature for coupling the shaft to a surgical handpiece;

Figures 2 and 2A are enlarged side views of a bur head shaped in accordance with this invention;

Figure 3 is a front view, looking proximally rearward, of the bur head of Figure 2;

Figure 4 is a cross sectional view of the bur head of Figure 2 taken along line 4-4 of Figure 2A;

Figure 5 is a cross sectional view of the bur head of Figure 2 taken along line 5-5 of Figure 2A; and

Figure 6 is a cross sectional view of the bur head of Figure 2 taken along line 6-6 of Figure 2A;

Figures 7 and 7A are side.views of an alternative bur head constructed in accordance with this invention;

Figure 8 is a front view, looking proximally rearward, of the bur head of Figure 7;

Figure 9 is a cross sectional view of the bur head of Figure 7 taken along line 9-9 of Figure 7A;

Figure 10 is a cross sectional view of the bur head of Figure 7 taken along line 10-10 of Figure 7A;

Figure 11 is a cross sectional view of the bur head of Figure 7 taken along line 11-11 of Figure 7A;

Figure 12 is a side view of a second alternative bur head constructed in accordance with this invention;

Figure 13 is a cross section view of the bur head of Figure 12 taken along line 13-13;

Figure 14 is a cross section view of the bur head of Figure 12 taken along line 14-14;

Figure 15 is a cross section view of the bur head of Figure 12 taken along line 15-15;

Figure 16 is a front view, looking proximally rearward of a third alternative bur constructed in accordance with this invention; and

Figure 17 is a cross sectional depiction of an alternative bur constructed in accordance with this invention.

DETAILED DESCRIPTION

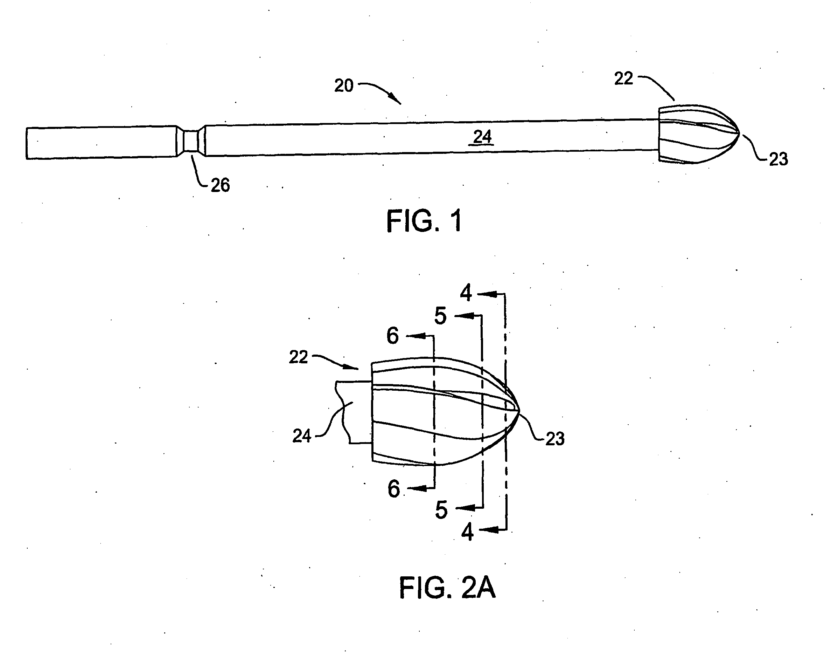

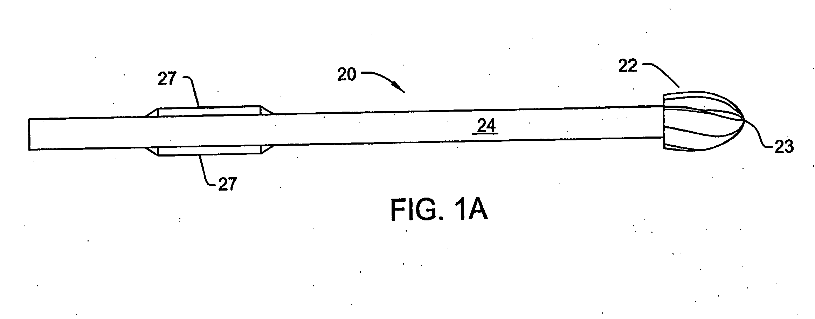

Figure 1 illustrates a surgical bur 20 constructed in accordance with this invention. Bur 20 has a head 22 that forms the distal end of the bur. ("Distal" it shall be understood, means towards the surgical site to which the bur is applied. "Proximal" means away from the surgical site.) Bur head 22 has a distal end tip 23 that is the most forward portion of the bur 20. A shaft 24 extends proximally rearward from the bur head 22.

The proximal end of the shaft 24 is provided with coupling features 26. The coupling features 26 are geometric features that facilitate the removable engagement of the shaft 24 to a coupling assembly integral with the rotating shaft of a powered surgical tool with which bur 20 is used (tool not illustrated.) The illustrated coupling features 26 are a set of planar faces recessed relative to the outer diameter of the shaft 24. One such geometry is described and illustrated in U.S. Patent No. 5,888,200, issued 30 March 1999, Multi-Purpose Surgical Tool System. An alternative geometry for coupling features 26 in the form of linearly aligned opposed concave surfaces is illustrated in U.S. Patent No. 6,562,055, issued 13 May 2003, Cutting Attachment For A Surgical Handpiece Designed To Be Selectively Coupled To The Handpiece . It should be appreciated that these two geometries of coupling features are exemplary, not limiting. In alternative versions of the invention, these coupling features may for example, be threading. Alternatively, as depicted in Figure 1A, tabs 27 that project outwardly from the outer surface of shaft 24 may function as the coupling features. The exact geometry of the coupling feature is not relevant to the structure of this invention.

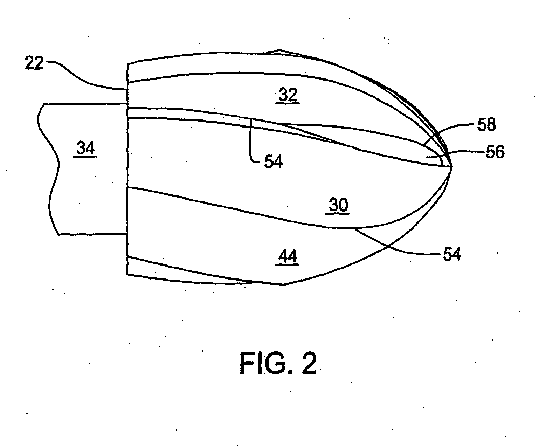

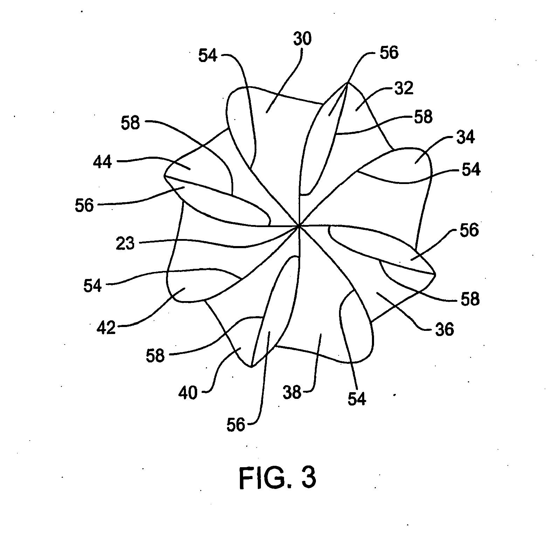

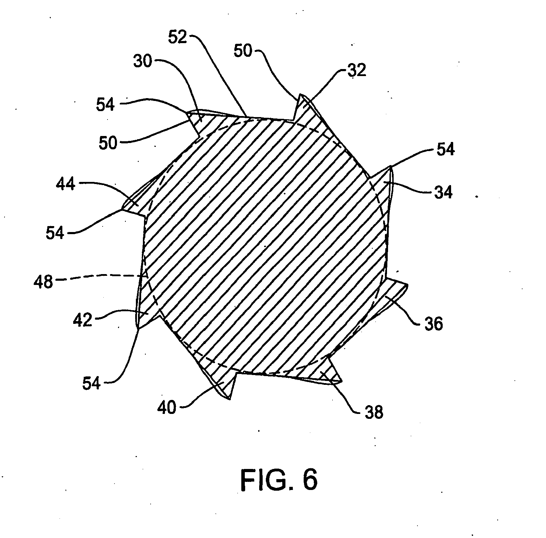

Bur head 22, as seen best in Figures 2, 2A and 3, is formed with a number of arcuately spaced flutes 30-44. Each flute 30-44, as seen by the cross sectional view of flute 30 in Figure 6, is formed by a rake surface 50 and a clearance surface 52. Rake surface 50 extends approximately radially from the longitudinal axis of the center core of the bur head 22. In the Figures, the outer perimeter of the center core is generally represented by dashed circle 48. It should be understood that, as the outer diameter of the bur changes along the length of the bur, the outer diameter of the center core changes. Each clearance surface 52 extends generally tangentially from the outer perimeter of the bur head center core. More specifically, each clearance surface 52 extends approximately tangentially away from the base of the rake surface 50 of the flute adjacent the flute formed by the clearance surface. Thus, the clearance surface 52 of flute 30 extends from the position along the perimeter of the bur head core from which rake surface 50 of flute 32 extends.

The rake surface 50 and clearance surface 52 that form an individual flute meet to forming a cutting edge 54. The bur cutting edges 54 are the edges of the bur head 22 that perform the cutting when the bur 20 is applied to a surgical surface.

As seen best by Figure 3, bur head 22 of this invention, flutes 30, 34, 38 and 42 are formed so that their rake and clearance surfaces 50 and 52, respectively, meet to form cutting edges 54 that start at a location relatively close to the distal end tip 23 of the bur head.

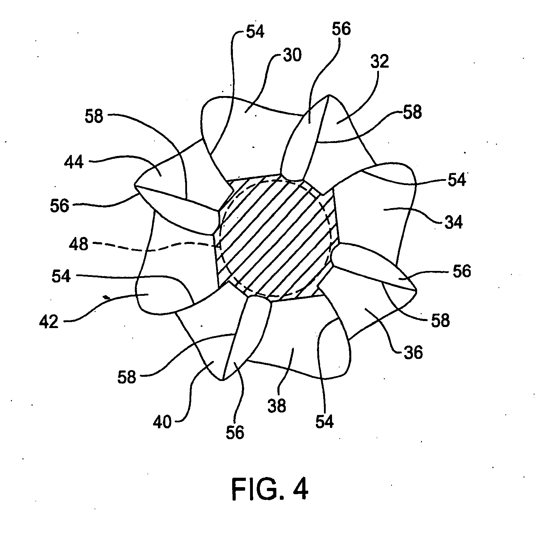

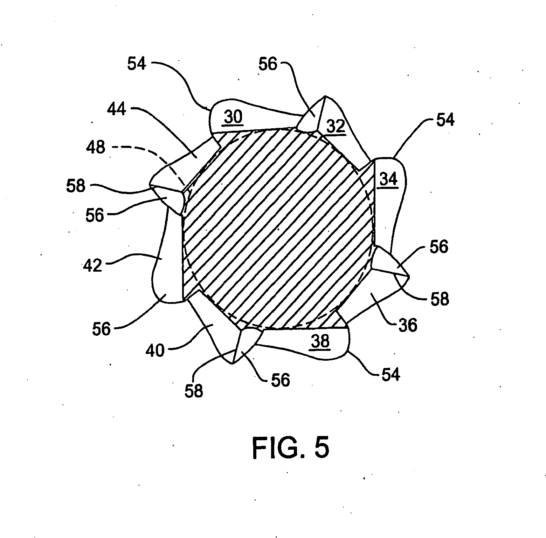

Flutes 32, 36, 40 and 44 are formed so that, at the distal ends of the flutes, chamfer surfaces 56, angled from the rake surfaces 50, extend between the rake surfaces 50 inwardly towards and to the clearance surfaces 52. Flutes 32, 36, 40 and 44 thus have chamfer edges 58 that are the edge surfaces along the interfaces between the clearance surfaces 54 and chamfer adjacent chamfer surfaces 56. As seen by Figures 2, 4, 5 and 6, chamfer surfaces 56 are formed on flutes 32, 36, 40 and 44 to end at the points along the flutes where the flutes have the largest outer diameter relative to the longitudinal center axis of the bur 20.

Bur head 22 may be formed by first shaping the head to provide eight (8) identical flutes that extend the full length of the head from the distal end tip to the shaft. Then portions of flutes 32, 36, 40 and 44 are selectively removed to form chamfer surfaces 52. Grinding, electro-discharge machining or laser cutting or other machining methods may be employed to excise the material from flutes 32, 36, 40 and 44 to form chamfer surfaces 56.

Owing to the presence of the chamfer surfaces 56, at any location along the longitudinal axis of the bur head where some flutes have cutting edges 54 and other flutes chamfer edges 58, the chamfer edges 58 are closer to this axis than cutting edges 54. Thus, chamfer edges 58 do not cut the tissue against which the bur head 22 is applied.

When a surgeon applies a bur 20 to a surgical site, often the section of the bur adjacent the distal end tip 23 is the section of the bur head 22 that is pressed against the tissue to be excised. It is at this time the above-described geometry of the bur of this invention becomes advantageous. There are a reduced number of cutting edges 54 at the distal end tip 23. It is believed this reduces the extent to which forces generated as a result of regeneration of waviness excite the bur into chatter vibration. Moreover, since there are a reduced number of cutting edges 56 at the most distal section of the bur head 22, the interstitial gap between cutting edges is wider than it would be otherwise. The relatively large size of these gaps minimizes the extent to which excised tissue is trapped in these spaces. This reduces the extent to which tissue entrained in the inter-flute gaps imposes addition vibration-causing force on the bur head 22.

The reduction of the number of cutting edges also reduces the tooth passing frequency at the distal tip of the bur 20. This is the frequency at the cutting edges 54 press against the tissue being excised. This frequency, TPF, is calculated according to the formula:

$$TPF = [RPM \times No. \text{ of } CE]/60$$

Here, RPM is the revolutions per minute of the bur 20. Variable CE is the number of bur head 22 cutting edges at the position along the bur head at which the bur head is being applied to the tissue to be excised. With bur head 22 of this invention, since there are fewer cutting edges at the distal end of the bur head 22 than at more proximal locations, the tooth passing frequency at the distal end locations is less than the tooth passing frequency at the more proximal locations along the length of the bur head.

This reduction in distal end tooth passing frequency further reduces the chatter of bur 20 of this invention. This is because a further means of reducing chatter is to operate the bur at a speed so that the tooth passing frequency as closely as possible matches the chatter frequency. This frequency matching assists in the nulling of bur chatter vibration. By reducing the number of flute cutting edges 54 at the distal end of the bur head 22, it is more likely that when, this end of the bur head is pressed against tissue, the tooth passing frequency will more closely approximate the chatter vibration frequency.

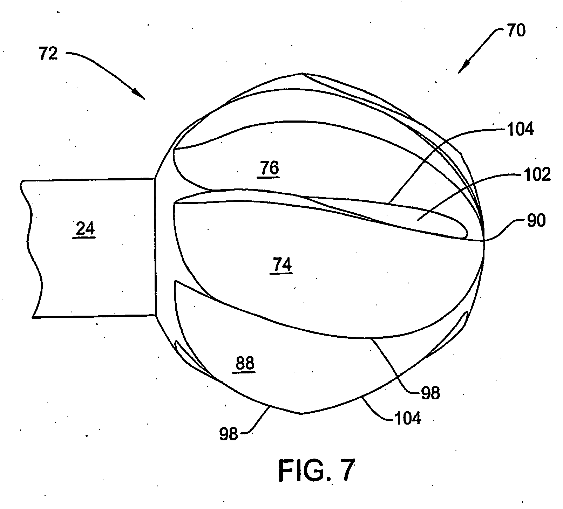

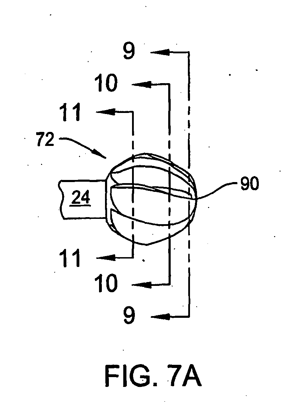

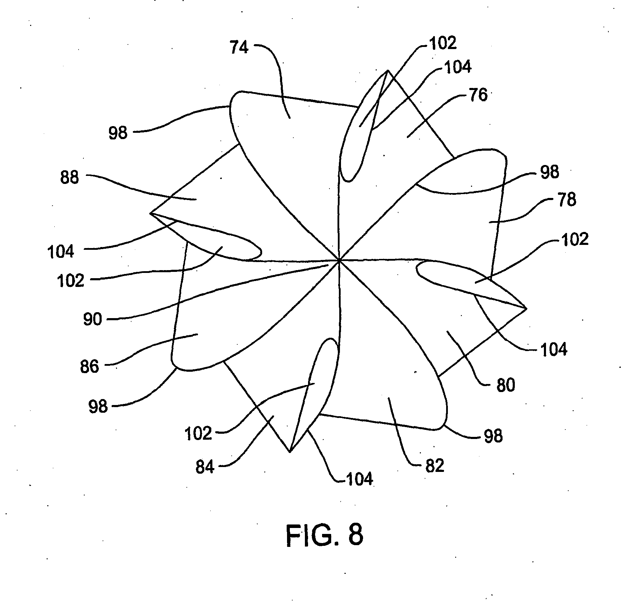

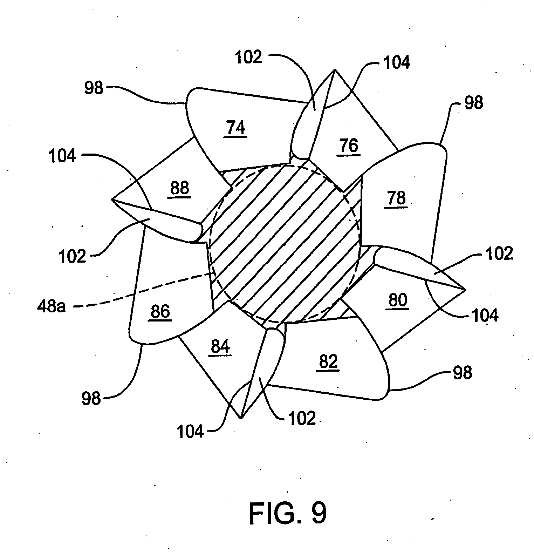

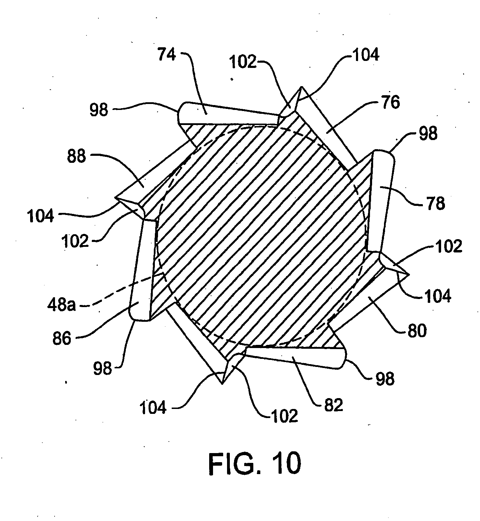

Bur head 22 of bur 20 has what is referred to as an acorn style head. As depicted by Figures 7, 7A and 8-11, this invention may be incorporated into bur heads having alternative shapes. Specifically, bur 70 of this version of the invention has what is referred to as a round or spherical head 72. As seen by reference to Figure 8, bur head 72 has flutes 74-88. Each of the flutes 74-88 originate at the distal end tip 90 of bur head 72.

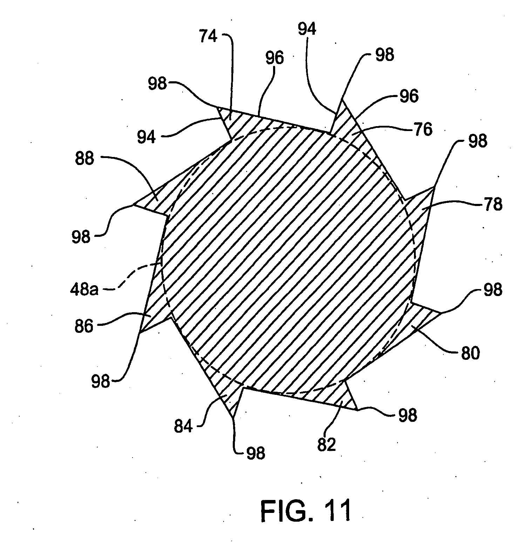

Each flute 74-88, as best seen in Figure 11, is formed with both a rake surface 94 and a clearance surface 96. Flutes 74, 78, 82 and 86 are formed to have cutting edges 98 that, like the flutes themselves, originate at the distal end tip 90 of the bur head.

Flutes 76, 80, 84 and 88 are formed with chamfer surfaces 102, best seen in Figures 8, 9 and 10, that start adjacent bur head distal end tip 90. Flutes 76, 80, 84 and 88 thus are formed with chamfer edges 104 where each clearance surface 96 meets the associated flute chamfer surface 102. Chamfer surfaces 102 are formed on flutes 76, 80, 84 and 88 so that chamfer edges 104 terminate and cutting edges 98 begin at the point where the bur head has its largest outer diameter.

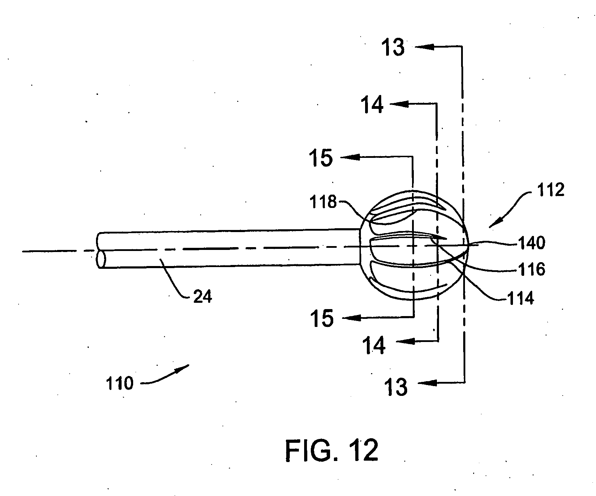

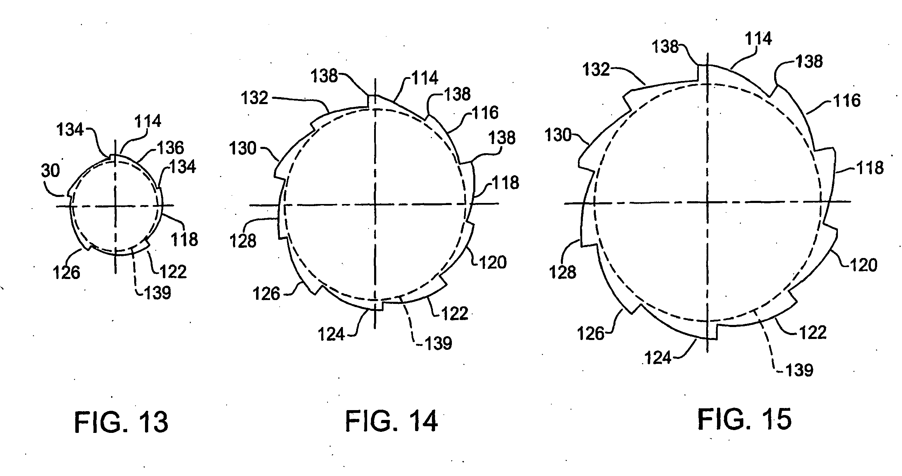

Figures 12-15 illustrate another bur 110 constructed in accordance with this invention. Bur 110 has a spherical bur head 112. Bur head 112 is shaped to have ten (10) flutes 114-132 best seen in Figure 15. Each flute 114-132 has a rake surface 134 and a clearance surface 136. The rake and clearance surfaces 132 and 134, respectively, of each flute 114-130 meet to form a cutting edge 138 that extends along the length of the flute.

Bur head 112 of this invention is formed so that the flutes originate from the core of the bur head, represented by dashed circles 139 at different positions along the length of the bur head. Figure 13, for example, illustrates the flutes present at a position adjacent distal end tip 140 of bur head 112. It can be seen here that only flutes 114, 118, 122, 126 and 130 have emerged from the core of bur head 112. Thus, clearance surface 136 of flute 130 emerges from the base of rake surface 134.

Extending proximally along the bur head 112, away from the distal end tip 140, flutes 116, 120, 124, 128 and 132 start to emerge from the core of bur head 112 as seen in Figure 14. Initially the rake surfaces 134 of flutes 116, 120, 124, 128 and 132 are shorter in length than the length of rake surfaces 134 of flutes 114, 118, 122, 126 and 130. Thus, the cutting edges 138 of flutes 116, 120, 124, 128 and 132 are spaced inwardly of the cutting edges of flutes 114, 118, 122, 126 and 130. Consequently, at the bur head position of line 14-14 of Figure 12, cutting edges 138 of flutes 116, 120, 124, 128 and 132 do not contribute to the cutting of the tissue to which the bur 110 is applied.

As seen by Figures 12 and 15, at the position along the length of the bur head 112 where the head is of largest diameter, flutes 116, 120, 124, 128 and 132 are fully emerged from the head core. All flutes 114-132 are of equal height relative to the perimeter of the bur head core. Thus the cutting edges 138 all contact, and therefore cut, the tissue against which this section of the bur is placed.

This version of the invention may be constructed by first forming bur head 112 so that all the flutes extend the full length of the bur head. Then material is removed from flutes 116, 120, 124, 128 and 132 so that the flutes emerge from the bur head core at a position proximal to the distal end tip 140. The means used to form chamfer surfaces 56 are employed to similarly shape flutes 116, 120, 124, 128 and 132.

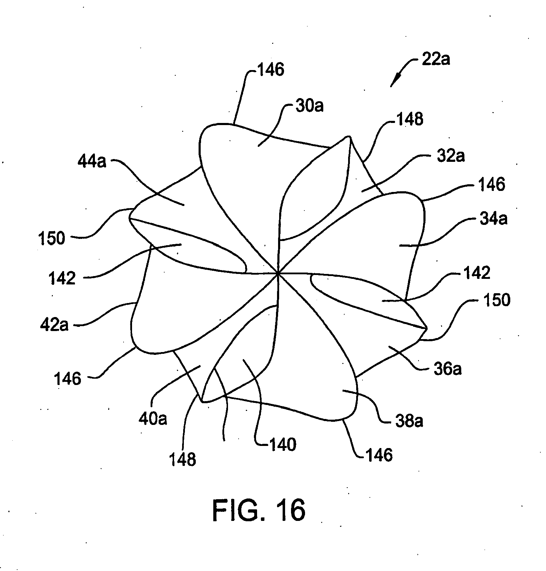

Figure 16 represents an alternative version of the invention of Figures 1-6. In this version of the invention bur head 22a is formed with flutes 30a-44a. Flutes 30a, 34a, 38a and 42a are not formed with chamfered surfaces. Flutes 32a and 40a are formed with chamfered surfaces 140. Flutes 36a and 44a are formed with chamfered surfaces 142. Flutes 32a and 40a are formed so that chamfered surfaces 140 extend a first distance proximally rearward along the length of bur head 22a. Flutes 36a and 44a are formed so that chamfered surfaces 142 extend a second distance proximally rearward along the length of the bur head 22a. This second distance is greater than the first distance chamfered surfaces 140 extend. Thus, bur head 22a of this embodiment of the invention is constructed to have a first set of cutting edges 146, where the clearance and rake surfaces of flutes 30a, 34a, 38a and 42a meet, which emerge from the core of the bur head at a first location along the length of the bur head. A second set cutting edges 148, where the clearance surfaces and chamfered surfaces 140 of flutes 32a and 40a meet, emerges from the core of the bur head at a second location along the length. A third set of cutting edges 150, where the clearance surfaces and chamfered surfaces of flutes 36a and 44a meet, emerges at a third location along the length of the bur head. This third location is, relative to the distal end tip, spaced further away than the second location.

It should be recognized that the foregoing are particular embodiments of the invention. Other versions of the invention may have features different from what has been described. For example, in the disclosed version of the invention, the flutes with the short length cutting edges are alternate with the flutes having the longer length cutting edges. This feature of the invention need not be incorporated in all versions of the invention. Thus, in some versions of the invention a bur head may be arranged so that two or more long length cutting edges are followed by one or more flutes with shorter length cutting edges.

Similarly, in some burs of this invention, each pair of adjacent flutes with long length cutting edges may be separated by two or more flutes with shorter length cutting edges. In regard to these versions of the invention, there may be some applications wherein flutes with shorter length cutting edges are adjacent, one flute may formed to have a cutting edge of a first length and a adjacent short length flute formed to have a cutting edge of a second length. Thus, a bur head may be constructed to have, in sequence: a flute with a cutting edge that extends from the distal end tip of the head; a flute with chamfer surface 140; and a flute with chamfer surface 142.

Further, there is no requirement that the bur heads of this invention be constructed so that the different length cutting surfaces are symmetrically arranged around the outer perimeter of the bur head. For example a bur head with eight (8) cutting edges may be constructed so that where the individual cutting edges are all present, the edges are spaced 45° apart. In this version of the invention, the first, second, fourth, sixth and seventh flutes are constructed so as to have cutting edges that start adjacent the bur head distal end tip. The third, fifth and eighth flutes are constructed to have cutting edges that start at a position spaced proximally to where the first set of flutes start.

In the above version of the invention it may be provided the bur shaft with a feature that offsets the asymmetric structure of the bur head. Such feature may include one or more tabs or fingers that are extend outwardly from the body of the shaft. These tabs are asymmetrically positioned to offset the asymmetrical loading. Alternatively, this feature may comprise one or more pins embedded in the shaft that are flush with the shaft. These pins are of different density than the material forming the bur shaft and are again asymmetrically positioned to offset the asymmetric loading.

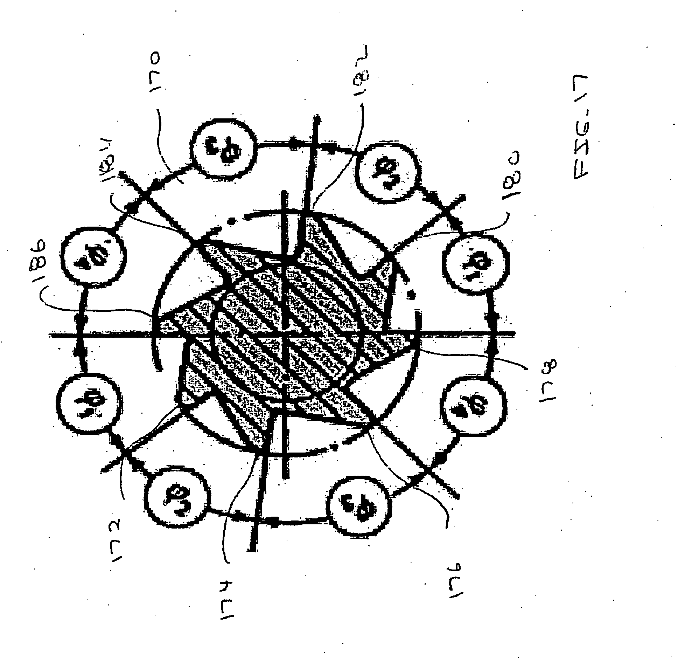

Figure 17 illustrates in cross section an alternative bur head 170 of this invention. Bur head 170 is shaped to have a number of arcuately spaced apart flutes 172-186. In this version of the invention flutes 172-186 may extend the complete length of the bur head 170, from the distal end tip to where the proximal end is joined to the shaft.

Bur head 170 is further formed so that around the circumference of the head, the flutes 172-186 are not equangularly spaced apart; the pitch angles between the adjacent flute pairs are different. For example, in the illustrated version of the invention flutes 172-186 and 178-186 are spaced apart angle $\varphi_1 = 38°$. Adjacent flutes 172-174 and 180-182 are spaced apart angle $\varphi_2 = 43°$. ˙Adjacent flutes 174-176 and 182-184 are spaced apart angle $\varphi_3 = 52°$. Adjacent flutes 176-178 and 184-186 are spaced apart angle $\varphi_4 = 47°$.

When the bur having this head geometry is employed, the between two adjacent pairs of flutes, for example between adjacent flutes 174-176 and 176-178, the time between when each flute in the pair strikes the tissue is different. Thus in the above example the time between when flute 176 strikes the bone after it is first struck by flute 174 is less than the time between when flute 178 strikes the bone after it is initially struck by flute 176. This variable periodicity between flute strikes is believed to reduce the extent the bur is forced into chatter-causing vibration.

This feature of the invention may or may not be incorporated into the versions of the invention with variable length cutting edges. Also, the bur head of this invention may be constructed so that not all adjacent pairs of the flutes have pitch angles that vary from each other. Thus, for example with a bur having eight flutes, there may be a first pitch angle between 6 of the adjacent flute pairs and a second pitch angle between two of the adjacent flute pairs

It should likewise be appreciated that the disclosed basic bur head shapes are exemplary, not limiting. In alternative versions of the invention, the bur heads may have alternative shapes including barrel head, conical, egg or drum shaped. Thus, there in some versions of the invention, the distal end tips of the bur heads may have profiles different than the curved convex profile of the illustrated embodiments.

Likewise, there is no requirement that, in all versions of the invention, the shorter length cutting edges emerge at the location where the bur heads reach there maximum diameter. The shorting length cutting edges are fully emerged at positions along the bur head distal to where the bur head has its larges diameter.

. Also, in some versions of the invention, the flutes with the shorter length cutting edges may have shorter length cutting edges by virtue of the cutting edges terminating at a position along the bur head spaced distally from the locations along which other cutting edges terminate. Some versions of the invention may be constructed so that, between flutes with cutting edges that extend the full length of the bur head, there are flutes with cutting edges that start a position proximal to the bur head distal end tip and terminate at a position distal to the proximal terminus of the bur head.

Further while two means of shaping the bur to provide cutting edges of different lengths are shown, in other versions of this invention may have different flute arrangements and flute shapes to provide the same structural features. Also, flutes of different shapes may be provided on a single bur. Thus, a single bur may have: a first set of flutes that are relatively long and shaped with relatively long cutting edges; a second set of flutes that are also relatively long and shaped with chamfered surfaces to have short cutting edges; and a third set of one or more flutes that are relatively short in length.

It is likewise understood that the shaft structure is not limited to what has been disclosed. The bur of this invention may have a tubular shaft. In these versions of the invention the shaft typically has an opening immediately proximal to the bur head. The opening functions as a port through which irrigating fluid is discharged or a suction is drawn. In these versions of the invention, the coupling feature of the bur is often a hub attached to the proximal open end of the shaft. The hub has both geometric features that facilitate the coupling of the bur to a drive handpiece and a port to establish fluid communication to a suction device or from a source of irrigating fluid.

Thus, it is an object of the appended claims to cover all such variations and modifications that come within the scope of this invention.

**Claims**

1. A surgical bur comprising:

   an elongated shaft (24) having opposed proximal and distal ends;
   a coupling feature (26, 27) associated with the proximal end of the shaft, the coupling feature shaped to engage a drive unit of a surgical handpiece so that the handpiece can rotate the shaft;
   a bur head (22, 22a, 72, 112) attached to the distal end of the shaft, the bur head having a body and a distal end and a plurality of arcuately spaced apart flutes (30-44, 30a-44a, 74-88, 114-132) that emerge from the body, each flute having a cutting edge, wherein the bur head is shaped so that a first set of flutes (30, 34, 38, 42) has cutting edges (54) that extend a first length along said bur head and a second set of said flutes (32, 36, 40, 44) has cutting edges that extend a second length, different from the first length, along the bur head, and wherein each flute has a rake surface (50, 94, 134 and a clearance surface (52, 96, 136) that meet to form the cutting edge (54, 98, 138) of the flute;

**characterized in that** the second set of flutes are formed with chamfered surfaces (56, 102, 140, 142) along a section thereof that extend from the rake surface to the clearance surface so that the second set of flutes do not have cutting edges along the sections thereof where the chamfered surfaces are present.

2. The surgical bur of Claim 1, wherein:

the bur head (22) is constructed so that the flutes forming the first and second set of flutes are interleaved with each other;
the first set of flutes (30, 34, 38, 42) are arranged so that the cutting edges of the flutes start at a first location proximal to the bur head body distal end;
the second set of flutes (32, 36, 40, 44) are arranged so that the cutting edges of the flutes start at a second location proximal to the bur head body distal end that is spaced further away from the first location from which the first set of flutes extend.

3. The surgical bur of Claims 1, or 2, wherein the bur head is constructed so that the flutes forming the first and second set of flutes alternate with each other around the circumference of the bur head.

4. The surgical bur of Claims 1, 2, or 3, wherein: the flutes forming the first and second sets are shaped so that the cutting edges thereof have a common proximal end terminus along the length of the bur head.

5. The surgical bur of Claims 1, 2, 3, or 4, wherein the bur head is formed so that the first set of flutes (114, 118, 122, 126, 130) emerge from the bur head (112) at a first location along the length of the bur head and the second set of flutes (116, 120, 124, 128, 132) emerge from the bur head at a second location along the length of the bur head.

6. The surgical bur of Claims 1, 2, 3, 4, or 5, wherein the bur head body is formed to have a third set of flutes (36a, 44a), the flutes forming the third set of flutes having cutting edges (150) extending a third length, different from the length of the cutting edges (146) of the first set of flutes (30a, 34a, 48a, 42) and the length of the cutting edges (148) of the second set of flutes (32a, 40a).

7. The surgical bur of Claims 1, 2, 3, 4, 5, or 6, wherein the first set of flutes are arranged symmetrically around the bur head and the second set of flutes are arranged symmetrically around the bur head.

8. The surgical bur of Claims 1, 2, 3, 4, 5, 6 or 7, wherein the bur head has shape selected from one of the following: acorn style; spherical; barrel; egg; and drum.

**Patentansprüche**

1. Chirurgischer Fräser, umfassend:

- einen länglichen Schaft (24) mit einander gegenüberliegenden proximalen und distalen Enden;
- eine dem proximalen Ende des Schafts zugeordnete Kupplungseinrichtung (26, 27), wobei die Kupplungseinrichtung ausgestaltet ist, mit einer Antriebseinheit eines chirurgischen Handstücks derart zusammenzuwirken, dass das Handstück den Schaft drehen kann;
- einen am distalen Ende des Schafts angebrachten Fräskopf (22, 22a, 72, 112), wobei der Fräskopf einen Körper, ein distales Ende und eine Vielzahl bogenförmig voneinander beabstandeter Schneiden (30-44, 30a-44a, 74-88, 114-132) aufweist, die von dem Körper ausgehen, wobei jede Schneide eine Schneidkante aufweist und wobei der Fräskopf derart geformt ist, dass eine erste Gruppe von Schneiden (30, 34, 38, 42) Schneidkanten (54) aufweist, die sich eine erste Länge entlang des Fräskopfes erstrecken, und eine zweite Gruppe von Schneiden (32, 36, 40) Schneidkanten aufweist, die sich entlang einer zweiten, sich von der ersten Länge unterscheidenden Länge entlang des Fräskopfes erstrecken, und wobei jede Schneide eine Wirkoberfläche (50, 94, 134) und eine Freioberfläche (52, 96, 136) aufweist, die zusammentreffen, um die Schneidkante (54, 98, 138) der Schneide zu bilden; **dadurch gekennzeichnet, dass** die zweite Gruppe von Schneiden abschnittsweise mit abgeschrägten Oberflächen (56, 102, 140, 142) ausgeformt ist, die sich von der Wirkoberfläche zur Freioberfläche derart erstrecken, dass die zweite Gruppe von Schneiden keine Schneidkanten entlang derjenigen Abschnitte aufweist, an denen die abgeschrägten Oberflächen vorhanden sind.

2. Chirurgischer Fräser nach Anspruch 1, wobei:

- der Fräskopf (22) derart ausgebildet ist, dass sich die Schneiden, welche die erste und zweite Gruppe von Schneiden bilden, miteinander verschachtelt sind;
- die erste Gruppe von Schneiden (30, 34, 38, 42) derart angeordnet ist, dass die Schneidkanten der Schneiden an einer ersten Stelle beginnen, die proximal zum distalen Ende des Körpers des Fräskopfes liegt;
- die zweite Gruppe von Schneiden (32, 36, 40, 44) derart angeordnet ist, dass die Schneidkanten der Schneiden an einer zweiten Stelle beginnen, die proximal zum distalen Ende des Körpers des Fräskopfes und weiter entfernt von der ersten Stelle, von der aus sich die erste Gruppe von Schneiden erstreckt, liegt.

3. Chirurgischer Fräser nach Anspruch 1 oder 2, wobei der Fräskopf derart ausgebildet ist, dass die Schneiden, welche die erste und zweite Gruppe von Schneiden bilden, einander um den Umfang des Fräskopfes herum abwechseln.

4. Chirurgischer Fräser nach einem der Ansprüche 1, 2 oder 3, wobei die Schneiden, welche die erste und zweite Gruppe bilden, derart geformt sind, dass ihre Schneidkanten einen gemeinsamen proximalen Endpunkt entlang der Länge des Fräskopfes aufweisen.

5. Chirurgischer Fräser nach einem der Ansprüche 1, 2, 3 oder 4, wobei der Fräskopf derart ausgeformt ist, dass die erste Gruppe von Schneiden (114, 118, 122, 126, 130) von dem Fräskopf (112) von einer ersten Stelle entlang der Länge des Fräskopfes ausgeht und die zweite Gruppe von Schneiden (116, 120, 124, 128, 132) von dem Fräskopf von einer zweiten Stelle entlang der Länge des Fräskopfes ausgeht.

6. Chirurgischer Fräser nach einem der Ansprüche 1, 2, 3, 4 oder 5, wobei der Körper des Fräskopfes aufgeformt ist, eine dritte Gruppe von Schneiden (36a, 44a) aufzuweisen, wobei die Schneiden, welche die dritte Gruppe von Schneiden bilden, Schneidkanten (150) aufweisen, die sich entlang einer dritten Länge erstrecken, die von der Länge der Schneidkanten (146) der ersten Gruppe von Schneiden (30a, 34a, 48a, 42) und der Länge der Schneidkanten (148) der zweiten Gruppe von Schneiden (32a, 40a) unterschiedlich ist.

7. Chirurgischer Fräser nach einem der Ansprüche 1, 2, 3, 4, 5 oder 6, wobei die erste Gruppe von Schneiden symmetrisch um den Fräskopf herum angeordnet ist und die zweite Gruppe von Schneiden symmetrisch um den Fräskopf herum angeordnet ist.

8. Chirurgischer Fräser nach einem der Ansprüche 1, 2, 3, 4, 5, 6 oder 7, wobei der Fräskopf eine der folgenden Formen aufweist: eichelförmig, sphärisch, tonnenförmig, eiförmig oder trommelförmig.

**Revendications**

1. Fraise chirurgicale comprenant :

un arbre allongé (24) ayant des extrémités proximale et distale opposées ;
une caractéristique de couplage (26, 27) associée avec l'extrémité proximale de l'arbre, la caractéristique de couplage formée de manière à engager une unité d'entraînement d'une pièce à main chirurgicale de telle sorte que la pièce à main peut faire tourner l'arbre ;
une tête de fraise (22, 22a, 72, 112) attachée à l'extrémité distale de l'arbre, la tête de fraise ayant un corps et une extrémité distale et une pluralité de goujures (30-44, 30a-44a, 74-88, 114-132) espacées selon un arc qui émergent du corps, chaque goujure ayant un bord de coupe, dans laquelle la tête de fraise est formée de telle façon qu'un premier ensemble de goujures (30, 34, 38, 42) ont des bords de coupe (54) qui s'étendent sur une première longueur le long de ladite tête de fraise et un deuxième ensemble desdites goujures (32, 36, 40, 44) ont des bords de coupe qui s'étendent sur une deuxième longueur, différente de la première longueur, le long de la tête de fraise, et dans laquelle chaque goujure a une surface d'attaque (50, 94, 134) et une surface de dépouille (52, 96, 136) qui se rencontrent pour former le bord de coupe (54, 98, 138) de la goujure ;

**caractérisée en ce que** le deuxième ensemble de goujures sont formées avec des surfaces chanfreinées (56, 102, 140, 142) sur une section de celles-ci qui s'étend de la surface d'attaque jusqu'à la surface de dépouille de telle sorte que le deuxième ensemble de goujures n'ont pas de bord de coupe sur les sections de celles-ci où les surfaces chanfreinées sont présentes.

2. Fraise chirurgicale selon la revendication 1, dans laquelle :

la tête de fraise (22) est construite de telle sorte que les goujures formant le premier et le deuxième ensembles de goujures sont entrelacées les unes avec les autres ;
le premier ensemble de goujures (30, 34, 38, 42) sont agencées de telle sorte que les bords de coupe des goujures commencent en un premier emplacement proximal par rapport à l'extrémité distale du corps de tête de fraise ;
le deuxième ensemble de goujures (32, 36, 40, 44) sont agencées de telle sorte que les bords de coupe des goujures commencent en un deuxième emplacement proximal par rapport à l'extrémité distale du corps de tête de fraise qui est espacé plus loin du premier emplacement à partir duquel le premier ensemble de goujures s'étendent.

3. Fraise chirurgicale selon la revendication 1, ou 2, dans laquelle la tête de fraise est construite de telle sorte que les goujures formant le premier et le deuxième ensemble de goujures alternent les unes avec les autres autour de la circonférence de la tête de fraise.

4. Fraise chirurgicale selon la revendication 1, 2, ou 3, dans laquelle : les goujures formant les premières et deuxièmes goujures sont formées de telle sorte que les bords de coupe de celles-ci ont une terminaison d'extrémité proximale commune sur la longueur de la tête de fraise.

5. Fraise chirurgicale selon la revendication 1, 2, 3, ou 4, dans laquelle la tête de fraise est formée de telle sorte que le premier ensemble de goujures (114, 118, 122, 126, 130) émergent de la tête de fraise (112) en un premier emplacement sur la longueur de la tête de fraise et le deuxième ensemble de goujures (116, 120, 124, 128, 132) émergent de la tête de fraise en un deuxième emplacement sur la longueur de la tête de fraise.

6. Fraise chirurgicale selon la revendication 1, 2, 3, 4 ou 5, dans laquelle le corps de tête de fraise est formé de manière à avoir un troisième ensemble de goujures (36a, 44a), les goujures formant le troisième ensemble de goujures ayant des bords de coupe (150) s'étendant sur une troisième longueur, différente de la longueur des bords de coupe (146) du premier ensemble de goujures (30a, 34a, 48a, 42) et de la longueur des bords de coupe (148) du deuxième ensemble de goujures (32a, 40a).

7. Fraise chirurgicale selon la revendication 1, 2, 3, 4, 5 ou 6, dans laquelle le premier ensemble de goujures sont agencées symétriquement autour de la tête de fraise et le deuxième ensemble de goujures sont agencées symétriquement autour de la tête de fraise.

8. Fraise chirurgicale selon la revendication 1, 2, 3, 4, 5, 6 ou 7, dans laquelle la tête de fraise a une forme choisie parmi l'une des suivantes : style de gland ; sphérique, cylindre ; oeuf ; et tambour.

FIG. 1

FIG. 2A

22

23

20

24

FIG. 1A

27

27

11

FIG. 2

FIG. 3

FIG. 4

FIG. 5

EP 1 903 954 B1

FIG. 6

FIG. 7

FIG. 7A

**FIG. 8**

FIG. 9

EP 1 903 954 B1

FIG. 10

FIG. 11

FIG. 12

FIG. 13          FIG. 14          FIG. 15

EP 1 903 954 B1

FIG. 16

FIG-17

**EP 1 903 954 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 342881 A
- DE 19826276 C1
- DE 19718938 C1
- US 5888200 A
- US 6562055 B